# EUROPÄISCHE PATENTANMELDUNG

(11) **EP 3 219 386 A1**
(43) Veröffentlichungstag der Anmeldung: **20.09.2017**
(21) Anmeldenummer: 16160200.8
(22) Anmeldetag: 14.03.2016
(51) Int. Cl.: B01J 23/887, B01J 37/10, B01J 37/00, C07C 45/35, C07C 253/24, C07C 253/26, B01J 23/89, B01J 27/00, B01J 35/00, B01J 27/057, B01J 27/192, B01J 37/02, B01J 35/10, B01J 37/08

(54) **VERFAHREN ZUR HYDROTHERMALEN HERSTELLUNG VON MOLYBDÄN-BISMUT-COBALT-EISEN-MISCHOXIDKATALYSATOREN**

(71) Anmelder: Evonik Degussa GmbH, 45128 Essen (DE)
(72) Erfinder: Sprenger, Paul, 76131 Karlsruhe (DE); Grundwaldt, Jan-Dierk, 76297 Stutensee (DE); Kleist, Wolfgang, 76149 Karlsruhe (DE); Fischer, Achim, 63773 Goldbach (DE)

(57) **Zusammenfassung**

Die vorliegende Erfindung betrifft ein Verfahren zur Herstellung von Molybdän-Bismut-Cobalt-Eisen-basierten Mischoxidkatalysatoren mittels Hydrothermalsynthese, wobei die Hydrothermalsynthese mit einer wässrigen Lösung und/oder einer wässrigen Suspension von Vorläuferverbindungen der im herzustellenden Mischoxidkatalysator enthaltenen Elemente durchgeführt, deren pH auf einen Wert von ca. 7 eingestellt wurde. Ferner betrifft die vorliegende Erfindung auch die nach diesem Verfahren erhältlichen Mischoxidkatalysatoren und ihre Verwendung in der Partialgasphasenoxidation von Olefinen.

## Beschreibung

Die vorliegende Erfindung betrifft ein Verfahren zur Herstellung von Multielementoxidkatalysatoren mittels Hydrothermalsynthese, die nach diesem Verfahren erhaltenen oder erhältlichen Multielementoxidkatalysatoren und ihre Verwendung in der Partialgasphasenoxidation von Olefinen.

Eingesetzt werden Multielementoxidkatalysatoren, insbesondere solche auf Basis von Molybdän- und Bismutoxiden, in der industriellen Herstellung von Acrolein und Acrylsäure bzw. Methacrolein und Methacrylsäure durch Partialgasphasenoxidation von Propen bzw. iso-Buten (2-Methyl-1-propen) oder tert-Butanol (2-Methyl-2-propanol) sowie von Acrylnitril durch Ammonoxidation bzw. Ammoxidation von Propen. Seit der Entwicklung von Bismutmolybdat-Katalysatoren für diese Umsetzungen durch das Unternehmen Standard Oil of Ohio (kurz Sohio) im Jahr 1959 haben diese Mischoxid- bzw. Multielementoxidkatalysatoren große Aufmerksamkeit erregt, und ihre katalytischen Eigenschaften wurden daher eingehend untersucht. Diese Untersuchungen betreffen den Einfluss weiterer Elemente zusätzlich zu Bismut und Molybdän auf Ausbeute und Selektivität für die Bildung von Acrolein bzw. Acrylnitril sowie die Stabilität des betreffenden Katalysators in der Reaktion. Ein Ergebnis dieser Untersuchungen war, dass unabhängig von weiteren Elementen die Oberflächenschicht dieser Multielementoxidkatalysatoren immer auf Oxiden von Bismut und Molybdän basiert. Daher werden diese Elemente als die aktiven Elemente dieser Katalysatoren betrachtet.

Typischerweise werden Multielementoxidkatalysatoren mittels der sogenannten Copräzipitation hergestellt, bei der Vorläuferverbindungen der Elemente des herzustellenden Katalysators in Wasser aufgelöst, anschließend aus dieser Lösung eine katalytische Aktivmasse mit diesen Elementen ausgefällt und diese Aktivmasse anschließend getrocknet und calciniert wird. Daneben werden Multielementoxidkatalysatoren, allerdings in deutlich geringerem Umfang, auch durch Festkörpersynthesen, Sol-Gel-Synthesen und Sprühtrocknung von Lösungen mit Vorläuferverbindungen des herzustellenden Multielementoxidkatalysators hergestellt. Der aus diesen Synthesen erhaltene Multielementoxidkatalysator sollte eine möglichst große Oberfläche aufweisen, um eine hohe Aktivität in der katalysierten Reaktion gewährleisten zu können. In der Regel wird eine hohe Aktivität bei Multielementoxidkatalysatoren dadurch erzielt, indem die Katalysatoren als kristalline Materialien vorliegen. Zur Bereitstellung kristalliner Materialien mittels der vorstehend genannten Synthesen sind jedoch immer hohe Temperaturen, insbesondere Temperaturen von mehr als 400°C, erforderlich, sei es um die Ausgangsverbindungen überhaupt erste zu den gewünschten Multielementoxiden umsetzen zu können, beispielsweise die Überführung von Nitraten zu den entsprechenden Oxiden, oder zur abschließenden Calcinierung des Katalysators. Derart hohe Temperaturen bewirken allerdings eine Verschlechterung der katalytischen Leistungsfähigkeit der resultierenden Phase des Mischoxids. Es wird vermutet, dass dieser Effekt auf eine Anreicherung von Bismut in der Katalysatoroberfläche zurückzuführen ist. Ein weiteres Problem der gängigen mittels Copräzipitation hergestellten Multielementoxidkatalysatoren besteht in ihrer zeitlich begrenzten Einsatzdauer. Innerhalb dieser zeitlich begrenzten Einsatzdauer altern die Katalysatoren, das heißt sie verfügen nach einer bestimmten Zeit nicht mehr über die gleiche katalytische Aktivität wie zu Beginn ihres Einsatzes in der katalysierten Reaktion. In diesem Fall muss das gealterte bzw. verbrauchte Katalysatorfestbett gegen frischen Katalysator ausgetauscht werden.

Die Alterung eines Mischoxid- bzw. Multielementoxidkatalysators mit einer Molybdän in oxidiertem Zustand enthaltenden Aktivmasse wird insbesondere dann beobachtet, wenn dieser Katalysator in einer Partialgasphasenoxidation eingesetzt wird, bei der Wasserdampf anwesend ist bzw. entsteht. In den meisten Fällen entsteht der Wasserdampf bei der Partialgasphasenoxidation. Es wird vermutet, dass der bei der partiellen Gasphasenoxidation anwesende bzw. entstehende Wasserdampf mit dem in der Aktivmasse des Katalysators in oxidierter Form vorliegenden Molybdän das leicht flüchtige MoO₂(OH)₂ bildet. In der Fachliteratur wird daher die Auffassung vertreten, dass während des Langzeitbetriebs einer heterogen katalysierten Gasphasenoxidation Molybdän kontinuierlich als MoO₂(OH)₂aus der Aktivmasse des Multielementoxidkatalysators heraussublimiert (vgl. Investigations of the mechanism and kinetics leading to a loss of molybdenum from bismuth molybdate catalysts, L. Zhang et al., Catalysis A: General (1994), 117, 163-171). Am stärksten ist der Verlust an der Aktivkomponente Molybdän in Mischoxidkatalysatoren insbesondere im Bereich der sogenannten Heißpunkte bzw. hot spots des Katalysatorfestbetts, also dort wo die Wärmentwicklung der am Katalysatorfestbett in Strömungsrichtung des Reaktionsgasgemisches ablaufenden Reaktion am stärksten ist. Gegebenenfalls weist ein Katalysatorfestbett mehrere sogenannte Heißpunkte auf. Nach Durchschreiten dieser Bereiche sinkt auch die Temperatur des Katalysatorfestbetts und seiner Umgebung in Strömungsrichtung der Gase deutlich, so dass sich Molybdänoxid als dünner Belag bzw. Film an den kälteren Stellen der Innenwand des Reaktionsrohres wieder niederschlägt (vgl. EP 2 155 376 A1). Nachdem ein Bereich des Katalysatorfestbetts an der Aktivkomponente Molybdän ausgeblutet ist, wandert ein Heißpunkt entlang des Katalysatorfestbetts weiter in Bereiche, in denen die Temperatur des Katalysatorfestbetts in Strömungsrichtung der Gase zuvor niedriger war und die daher noch nicht an Molybdän ausgeblutet sind. Auch diese Bereiche bzw. Zonen verarmen dann schrittweise an Molybdän. Aufgrund des zonenweisen Verarmens bzw. Ausblutens des Katalysators an der Aktivkomponente Molybdän wird dieser Vorgang auch als Zonenalterung (im Englischen band aging) bezeichnet.

Mit zunehmender Betriebsdauer des Multielementoxidkatalysators nimmt daher nicht nur der Anteil an der Komponente Molybdän im Katalysator ab sondern auch die Aktivität des Katalysators für die zu katalysierende Reaktion. Dies macht sich in einem verminderten Umsatz für die zu oxidierende Komponente, beispielsweise Propen, bemerkbar. Dem Umsatzrückgang kann durch Erhöhung der Temperatur des Salzbads, das die den Katalysator enthaltenden Rohre des Reaktors umgibt, entgegengewirkt werden. Hierbei wird die für einen spezifischen Umsatz erforderliche Temperatur des Salzbads auf einen Wert erhöht, der bei ansonsten unveränderten Reaktionsbedingungen denselben Umsatz für die zu oxidierende Verbindung bei einem einmaligen Durchgang durch den Reaktor erzielt wie vor der Abnahme der Aktivität des Katalysators. Die Erhöhung der Temperatur des Salzbades, um der Deaktivierung der Aktivmasse eines Molybdän in oxidierter Form enthaltenden Mischoxidkatalysators entgegenzuwirken, ist jedoch kontraproduktiv für die Lebensdauer des betreffenden Mischoxidkatalysators. Denn höhere Temperaturen im Katalysatorfestbett beschleunigen die Alterung bzw. Deaktivierung des Katalysators, was frühzeitig entweder den vollständigen oder zumindest den teilweisen Ersatz des gealterten Katalysatorfestbetts durch frischen Katalysator erforderlich macht. Dies führt zwangsläufig zu häufigeren Wechseln des Katalysatorfestbetts und häufigeren Produktionsstillständen. Neben dem damit verbundenen Produktionsausfall stellen auch die häufigere Entsorgung des verbrauchten Katalysators sowie die häufigere Herstellung von neuem Katalysator eine erhebliche finanzielle Belastung dar.

Alternativ zu den gängigen Herstellungsverfahren können Mischoxidkatalysatoren auf Basis von Bismutmolybdaten auch mittels der Hydrothermalsynthese hergestellt werden. Im Vergleich zu den vorstehend genannten Herstellungsverfahren sind die Reaktionsbedingungen bei der Hydrothermalsynthese im Allgemeinen deutlich weniger drastisch. Die Hydrothermalsynthese ermöglicht daher einen praktischen und gut reproduzierbaren Zugang zu Materialien mit hoher Reinheit, kontrollierter Morphologie und hoher Kristallinität. Bisher wurden mittels Hydrothermalsynthese allerdings nur Bismutmolybdate, also nur binäre Systeme hergestellt. Auch wenn mit Bismutmolybdaten gute Selektivitäten für die Bildung von Acrolein in der katalysierten Partialgasphasenoxidation von Propen erzielt werden, eignen sich diese Systeme dennoch nicht für einen großtechnischen Einsatz. Denn die mit Bismutmolybdaten in dieser Reaktion erzielten Propen-Umsätze betragen nicht mehr als ca. 15%. Bei derart niedrigen Propenumsätzen müssen dann allerdings besonders große Menge an nichtumgesetzten Propen recycliert werden. Dadurch steigen sowohl der Energieaufwand als auch der Investitionsaufwand immens an, was eine großtechnische Umsetzung uninteressant macht.

Ferner enthalten Industriell eingesetzte Mischoxidkatalysatoren für die Oxidation von Propen auch noch weitere Elemente als Molybdän und Bismut. Wechselt man bei der Hydrothermalsynthese eines Mischoxidkatalysators von einem binären System wie Bismutmolybdat zu einem komplexeren Mehrkomponentensystem, das neben Bismut und Molybdän auch Cobalt und Eisen enthält, hat dies unmittelbare Auswirkung darauf, welche Phasen gebildet werden, welche Mengen an katalytisch aktiven Elementen in diese Phasen eingebaut werden und wie groß die Oberfläche der Phasen ist.

Da ein hinsichtlich Aktivität und Selektivität optimierter Multielementoxidkatalysator eine Kombination aus einer erhöhten Oberfläche, geeigneten Kristallphasen und einer maßgeblichen Menge katalytisch aktiver Elemente darstellt, bestand daher ein Bedarf an einem Verfahren zur Bereitstellung entsprechender Multielementoxidkatalysatoren mit hoher Kristallinität. Erfindungsgemäß wird diese Aufgabe dadurch gelöst, indem eine Hydrothermalsynthese von mit einer wässrigen Lösung und/oder einer wässrigen Suspension von mindestens vier Metall-Vorläuferverbindungen, insbesondere mindestens vier Übergangsmetall-Vorläuferverbindungen, der im herzustellenden Multielementoxidkatalysator enthaltenen Metalle durchgeführt wird, deren pH auf einen Wert zwischen ca. 6 und ca. 8 eingestellt wurde.

Gegenstand der vorliegenden Erfindung ist daher ein Verfahren zur Herstellung eines Multielementoxidkatalysators der allgemeinen Formel (I)

MoₐBi_{b}Co_{c}Fe_{d}NiₑX_{f}X'_{g}X"ₕX'"ᵢX""ⱼOₓ (I) mit
- X: = W und/oder P,
- X': = Li, Na K, Rb, Cs, Mg, Ca, Sr und/oder Ba,
- X": = Ce, Mn, Cr und/oder V,
- X'": = Nb, Se, Te, Sm, Gd, La, Y, Pd, Pt, Ru, Ag und/oder Au,
- X"": = Si, Al, Ti und/oder Zr,
und
- a: = 12,
- b: = 1 bis 4,
- c: = 4 bis 10,
- d: = 1 bis 4,
- e: = 0 bis 4,
- f: = 0 bis 5,
- g: = 0 bis 2,
- h: = 0 bis 5,
- i: = 0 bis 2,
- j: = 0 bis 800,
und
x = eine Zahl, die von der Wertigkeit und Häufigkeit der von Sauerstoff verschiedenen Elemente bestimmt wird, umfassend die Schritte
a) Bereitstellen einer Mischung aus einer wässrigen Lösung und/oder einer wässrigen Suspension von Vorläuferverbindungen der im herzustellenden Multielementoxidkatalysator enthaltenen Elemente in der zur Erzielung der Stöchiometrie gemäß der allgemeinen Formel erforderlichen Menge,
b) Einstellen der aus Schritt a) erhaltenen Mischung auf einen pH-Wert zwischen 6 +/- 0,5 und 8 +/- 0,5,
c) Umsetzen der Ausgangsverbildungen unter im Wesentlichen solvothermalen Reaktionsbedingungen unter Bildung eines Multielementoxidkatalysators der allgemeinen Formel (I), und
d) Abtrennen des Multielementoxidkatalysators von der wässrigen Lösung und/oder Suspension.

Im Rahmen der vorliegenden Erfindung werden die Bezeichnungen Multielementoxidkatalysator und Mischoxidkatalysator gleichbedeutend verwendet und bezeichnen einen Katalysator der zumindest aus den erfindungsgemäßen Elementen Molybdän, Bismut, Cobalt und Eisen besteht.

Das erfindungsgemäße Verfahren dient daher vorzugsweise zur Herstellung eines Multielementoxidkatalysator der allgemeinen Formel (I) BiₐMo_{b}Co_{c}Fe_{d}Oₓ mit a = 1 bis 4, b = 12, c = 4 bis 10 und d = 1 bis 4.

Insbesondere wird durch das erfindungsgemäße Verfahren ein Multielementoxidkatalysator der allgemeinen Formel (I) BiₐMo_{b}Co_{c}Fe_{d}Oₓ mit den Bedeutungen a = 1 bis 2, b = 12, c = 5 bis 8 und d = 2 bis 3 erhalten.

Im Rahmen der vorliegenden Erfindung wird der Begriff Hydrothermalsynthese gemäß dem allgemeinen Fachwissen des Fachmanns verwendet und bezeichnet eine in Wasser bei einer Temperatur oberhalb von 100°C und einem Druck oberhalb von 1 bar durchgeführte heterogene Reaktion. Diese Reaktionsbedingungen, also Temperaturen und Drücke oberhalb von 100°C bzw. oberhalb von 1 bar werden auch als solvothermale oder hydrothermale Reaktionsbedingungen bezeichnet. Bei Hydrothermalsynthesen ist in der Regel die Verwendung von Autoklaven erforderlich. Sie dienen zum Schutz der Reaktionsgefäße, wobei häufig der Autoklav selbst das Reaktionsgefäß ist. Der Druck im Reaktionsgefäß wird über die Temperatur geregelt - bei Verwendung von Reaktionsmedium führen Temperaturen von 200°C in einem nach außen hin abgeschlossenen Reaktionsgefäß bereits zu Überdrücken von 800 mbar und mehr. Die Verwendung eines Autoklaven selbst als druckresistentem Reaktionsgefäß ermöglicht es die Hydrothermalsynthese über einen weiten Druck- bzw. Temperaturbereich durchführen zu können. Ferner hat ein Autoklav gegenüber anderen nach außen hin abgeschlossenen Reaktionsgefäßen wie beispielsweise Ampullen den Vorteil, dass dem Reaktionsmedium auch während der Reaktion kontinuierlich weitere leicht lösliche Komponente zugeführt werden können, beispielsweise Säuren, Basen oder andere komplexbildende Stoffe, die auch als Mineralisatoren bezeichnet werden. Bevorzugt wird daher das erfindungsgemäße Verfahren oder zumindest der Schritt c) des erfindungsgemäßen Verfahrens in einem Autoklaven durchgeführt.

Hinsichtlich der Vorläuferverbindungen der im herzustellenden Multielementoxidkatalysator enthaltenen Elemente unterliegt das erfindungsgemäße Verfahren prinzipiell keinen Einschränkungen. Vorteilhafterweise werden solche Vorläuferbindungen eingesetzt deren Anionen sich bei einer der Hydrothermalsynthese gegebenenfalls anschließenden thermischen Behandlung rückstandlos entfernen lassen. Bevorzugt werden daher Nitrate, Carbonate, Formiate, Oxalate oder ähnliche Verbindungen als Vorläuferverbindungen im erfindungsgemäßen Verfahren eingesetzt.

Im Rahmen der vorliegenden Erfindung umfasst der Ausdruck ein pH-Wert zwischen 6 +/- 0,5 und 8 +/-0,5 alle pH-Werte von einschließlich 5,5 bis einschließlich 8,5, die durch ganze und/oder reelle Zahlen wiedergegeben werden können, insbesondere die pH-Werte 5,5; 6; 6,5; 7; 7,5; 8 und 8,5.

Multielementkatalysatoren, die bei diesen pH-Werten mittels Hydrothermalsynthese hergestellt wurden, katalysieren die Oxidation von Propen zu Acrolein mit einer Selektivität von mindestens 50% bis zu ca. 82%.

Vorzugsweise wird der pH in Schritt b) des erfindungsgemäßen Verfahrens auf einen Wert zwischen 6 und 8 eingestellt, der also der Bedingung 6 ≤ pH ≤ 8 entspricht, insbesondere auf einen Wert, der größer als 6 und kleiner als 8 ist.

Indem der Schritt b) des erfindungsgemäßen Verfahrens bei einem noch engeren pH-Bereich durchgeführt wird, insbesondere bei einen pH-Wert von 7 +/- 0,5, wird nicht nur eine Selektivität für die Bildung von Acrolein auf Werte von mindestens 65% bis zu ca. 82% erreicht, sondern zudem auch der Propenumsatz auf Werte von bis zu ca. 65% gesteigert.

In einer Ausführungsform des erfindungsgemäßen Verfahrens wird daher in Schritt b) ein pH-Wert von 7 +/- 0,5 eingestellt.

Im Rahmen der vorliegenden Erfindung wurde gefunden, dass die Wahl des pH-Wertes in der Synthese die Zusammensetzung des Katalysators stark beeinflusst. Tendenziell haben steigende pH-Werte in der Hydrothermalsynthese eine deutliche Abnahme des Gehalts an der katalytisch aktiven Komponente Molybdän im hergestellten Katalysator zur Folge; gleichzeitig wird eine deutliche Zunahme des Anteils der Komponente Bismut beobachtet. In der Fachliteratur ist allgemein die Rolle von Molybdän als katalytisch aktive Komponente für die Gasphasenpartialoxidation anerkannt. Es sollte daher erwartet werden, dass der bei pH = 5 hergestellte Katalysator mit dem höchsten Molybdän-Anteil auch die besten katalytischen Eigenschaften in der Partialgasphasenoxidation von Propen aufweisen sollte. Untersuchungen haben jedoch gezeigt, dass der bei einem pH-Wert von 7 +/- 0,5 hergestellte erfindungsgemäße Katalysator sowohl die höchste Acrolein-Selektivität als auch den höchsten Propen-Umsatz zeigt.

Ferner wurde gefunden, dass der in Schritt b) des erfindungsgemäßen Verfahrens eingestellte pH-Wert auch einen Einfluss auf die spezifische Oberfläche der Katalysatoren hat: Mit steigendem pH-Wert nimmt auch die spezifische Oberfläche der Katalysatoren zu. Eine große Oberfläche eines Katalysators wird üblicherweise mit einer hohen katalytischen Aktivität korreliert. Man sollte daher erwarten, dass die bei höheren pH-Werten, pH = 8 oder mehr, hergestellten Katalysatoren die höchsten Propen-Umsätze in der Partialgasphasenoxidation von Propen zeigen sollten. Überraschenderweise wurde allerdings in Untersuchungen gefunden, dass der bei einem pH von 7 +/- 0,5 hergestellte Katalysator den höchsten Propen-Umsatz in der Partialgasphasenoxidation von Propen zeigt.

Ferner wurde außerdem gefunden, dass die Wärmetönung bei der katalysierten Reaktion für die bei pH = 7 hergestellten Multielementoxidkatalysatoren geringer ist als bei dem durch Copräzipitation hergestellten Vergleichskatalysator. Folglich wird ein entsprechender Katalysator beim Einsatz in der Partialgasphasenoxidation von Olefinen einer geringeren thermischen Belastung ausgesetzt. Dadurch verarmt der Katalysator beispielsweise nicht so schnell an der katalytisch aktiven Komponente Molybdän. In Summe wirkt sich die geringere thermische Belastung positiv auf die Lebensdauer des Katalysators aus.

Ein pH von 7 +/- 0,5 stellt daher eine besonders günstige Wahl für den pH-Wert in der Hydrothermalsynthese dar. Die Einstellung des pH auf 7 +/- 0,5 führt scheinbar zu einem Katalysator, der eine besonders gut Kombination von Molybdän-Gehalt und spezifischer Oberfläche aufweist, denn der unter diesen Bedingungen erhaltene Katalysator führt zu einer besonders guten Acrolein-Selektivität und gleichzeitig auch zu einem besonders guten Propen-Umsatz.

Gegebenenfalls erreichen die Vorläuferverbindungen der im herzustellenden Katalysator enthaltenen Elemente in reinem Wasser als solvothermalem Reaktionsmedium nicht die erforderliche Löslichkeit. Zur Bildung anderer, besser löslicher Komplexe der betreffenden Elemente als mit reinem Wasser wird daher der Lösung und/oder Suspension der Vorläuferverbindungen bevorzugt eine Säure, Base oder ein anderer komplexbildender Stoff hinzugegeben. Bezüglich der hinzuzugegebenen Säure, Base oder dem komplexbildenden Stoff unterliegt das erfindungsgemäße Verfahren grundsätzlich keinen Einschränkungen. Vorteilhafterweise wird die hinzuzugegebene Säure, Base oder der komplexbildender Stoff so gewählt, dass er sich nach der Hydrothermalsynthese mit geringem Aufwand rückstandlos entfernen lässt, beispielsweise durch einfaches Waschen oder durch Zersetzung bei erhöhten Trocknungstemperaturen. Als Hilfsstoffe in der Hydrothermalsynthese, auch als Mineralisatoren bezeichnet, kommen daher beispielsweise Salpetersäure, Essigsäure, Ameisensäure oder ähnliche Verbindungen zum Einsatz. Bevorzugt wird in Schritt a) des erfindungsgemäßen Verfahrens Salpetersäure zur Verbesserung der Löslichkeit der Vorläuferverbindungen hinzugegeben.

Zusätzlich zu den vorstehend genannten Säuren verhalten sich manche der Vorläuferverbindungen bzw. manche der in hydratisierter Form vorliegenden Metallkationen der Vorläuferverbindungen in einem wässrigen Medium als teilweise starke Säuren. Durch Zugabe einer geeigneten Base wird der gewünschte pH-Wert in Schritt b) des erfindungsgemäßen Verfahrens eingestellt. Hinsichtlich der Wahl dieser Base unterliegt das erfindungsgemäße Verfahren prinzipiell keinen Einschränkungen, solange gewährleistet ist, dass durch ihre Zugabe ein pH-Wert zwischen 6 +/- 0,5 und 8 +/- 0,5, bevorzugt ein pH-Wert von größer als 6 und kleiner als 8 und insbesondere ein pH-Wert von 7 +/-0,5, eingestellt werden kann und die zugegebene Base die Bildung des Multielementoxidkatalysator nicht stört. Bevorzugt wird im erfindungsgemäßen Verfahren zur Einstellung des gewünschten pH-Werts eine Base eingesetzt, die sich durch Waschvorgänge und/oder durch eine anschließende thermische Behandlung, insbesondere bei niedrigen Temperaturen, leicht von dem hergestellten Katalysator entfernen lässt. Bevorzugt wird eine komplexierende Base zur Einstellung des pH-Wertes in Schritt b) des erfindungsgemäßen Verfahrens eingesetzt. Ohne sich auf eine spezifische Theorie festlegen zu wollen wird vermutet, dass die Anwesenheit einer komplexierenden Base sich vorteilhaft auf die die katalytischen Eigenschaften des hergestellten Multielementoxidkatalysators auswirkt. Daher wird vorzugsweise eine stickstoffhaltige Base zur Einstellung des pH-Wertes in Schritt b) des erfindungsgemäßen Verfahrens eingesetzt. Bevorzugt handelt es sich bei der stickstoffhaltigen Base um Ammoniak oder ein primäres, sekundäres oder tertiäres aliphatisches C₁-C₄-Amin, beispielsweise um Methylamin, Ethylamin, Dimethylamin, Diethylamin, Trimethylamin oder Triethylamin. Bevorzugt wird eine wässrige Ammoniak-Lösung als Base verwendet, insbesondere eine wässrige Ammoniak-Lösung mit einer Konzentration von mindestens 20%, insbesondere mit einer Konzentration von 20% bis 30%, beispielsweise 25%.

Das Reaktionsgefäß, bevorzugt der Autoklav, wird für die Durchführung der Hydrothermalsynthese in einem Ofen platziert: Es kann sowohl isotherm als auch mit einem Temperaturgradienten gearbeitet werden. Vorzugsweise wird im Rahmen des erfindungsgemäßen Verfahrens isotherm gearbeitet, das heißt dass der befüllte Autoklav mitsamt seinem Inhalt nach seiner Versiegelung auf eine definierte Temperatur aufgeheizt wird, diese Temperatur für eine spezifische Zeitdauer gehalten wird und im Anschluss der Autoklav mitsamt seinem Inhalt über eine spezifische Zeitdauer auf die Ausgangstemperatur, bevorzugt Raumtemperatur, abgekühlt wird bzw. man den Autoklaven mitsamt Inhalt auf die Ausgangstemperatur, bevorzugt Raumtemperatur, abkühlen lässt. Diese Vorgehensweise ermöglicht es, das erfindungsgemäße Verfahren unter im Wesentlichen konstanten Reaktionsbedingungen durchzuführen.

In einer Ausführungsform des erfindungsgemäßen Verfahrens wird der Schritt c) bei einer Temperatur von mehr als 100°C bis 600°C durchgeführt. Bezogen auf Wasser als solvothermales Reaktionsmedium entspricht die Durchführung von Schritt c) bei diesen Temperaturen einem Innendruck im Autoklaven von mehr als 1 bar bis zu ca. 3.000 bar.

In einer bevorzugten Ausführungsform des erfindungsgemäßen Verfahrens wird der Schritt c) bei einer Temperatur von mehr als 100°C bis 400°C durchgeführt. Diese Reaktionstemperatur führen bei der Durchführung von Schritt c) mit Wasser als solvothermalem Reaktionsmedium zu einem Innendruck von mehr als 1 bar bis zu ca. 100 bar.

Im Rahmen der vorliegenden Erfindung umfasst die Angabe eine Temperatur von mehr als 100°C bis 600°C bzw. die Angabe eine Temperatur von mehr als 100°C bis 400°C sämtliche Werte von über 100°C bis einschließlich 600°C bzw. von über 100°C bis einschließlich 400°C, die durch ganze und reelle Zahlen wiedergegeben werden können.

Im Rahmen der vorliegenden Erfindung umfasst die Angabe ein Druck von mehr als 1 bar bis 3.000 bar bzw. die Angabe ein Druck von mehr als 1 bar bis 100 bar sämtliche Werte von über 1 bar bis einschließlich 3.000 bar bzw. von über 1 bar bis über 100 bar, die durch ganze und reelle Zahlen wiedergegeben werden können.

Hinsichtlich der Zeitdauer in der der Schritt c) durchgeführt wird unterliegt das erfindungsgemäße Verfahren keinen Einschränkungen, solange gewährleistet bleibt, dass das Verfahren einen Mischoxidkatalysator der erfindungsgemäßen Zusammensetzung ergibt.

In einer Ausführungsform des erfindungsgemäßen Verfahrens wird daher der Schritt c) für eine Zeitdauer von 6 +/- 0,5 Stunden bis zu 48 +/- 0,5 Stunden durchgeführt.

Vorteilhafterweise schließen sich dem erfindungsgemäßen Verfahren noch weitere Schritte der Nachbehandlung des aus dem erfindungsgemäßen Verfahren erhaltenen Multielementoxidkatalysators an. Insbesondere handelt es sich dabei um ein- oder mehrmaliges, bevorzugt mehrmaliges, Waschen des aus der Hydrothermalsynthese erhaltenen Multielementoxidkatalysators zur Entfernung von Rückständen aus der Synthese. Zum Waschen sind grundsätzlich alle Lösungsmittel geeignet, die Rückstände aus der Synthese in einem oder mehreren Waschgängen vollständig entfernen können und den synthetisierten Katalysator hinsichtlich seiner Struktur und Zusammensetzung unverändert lassen. Bevorzugt werden allerdings solche Lösungsmittel, die selber auch durch geringen Aufwand rückstandsfrei vom Katalysator entfernt werden können. Insbesondere solche Lösungsmittel, die durch Trocknung bei niedrigen Temperaturen, insbesondere bei Temperaturen unterhalb der für eine Calcinierung üblichen 400°C, entfernbar sind, werden zum Waschen bevorzugt eingesetzt. Beispielhafte Lösungsmittel zum Waschen sind Wasser und Aceton. Der aus der Synthese erhaltene Multielementoxidkatalysator wird dann beispielsweise ein- oder mehrmals, bevorzugt mehrmals, beispielsweise dreimal, mit Wasser und anschließend ein- oder mehrmals, bevorzugt mehrmals, beispielsweise dreimal, mit Aceton gewaschen. Im Anschluss wird der gewaschene Multielementoxidkatalysator getrocknet. Dies geschieht bevorzugt bei Temperaturen, die unterhalb der für eine Calcinierung üblichen Temperaturen von 400°C und mehr liegen. Bevorzugt reicht die Temperatur beim Trocknen des Multielementoxidkatalysators von 20°C bis maximal 390°C, bevorzugt reicht die Trocknungstemperatur von 20°C bis 350°C. Diese Temperaturen, die unter den für eine Calcinierung üblicherweise angewandten Temperaturen liegen, reichen bereits aus, damit sich Nitrate zersetzen. Die Dauer der Trocknung wird ausreichend lang gewählt, um sicherzustellen dass sämtliche Rückstände aus der Synthese und sämtliches Lösungsmittel aus dem Waschvorgang von dem Multielementoxidkatalysator vollständig entfernt wurden. Der Zeitpunkt an dem dies erreicht ist, kann durch Trocknen bis zum Eintreten einer Gewichtskonstanz beispielsweise mittels Thermogravimetrie bestimmt werden. Eine Dauer von bis zu 3 Tagen, insbesondere 12, 24, 36 oder 48 Stunden, reicht in der Regel selbst bei einer Temperatur von 20°C zur Trocknung eines gewaschenen Multielementoxidkatalysators aus. Im Gegensatz zu den nach den bisherigen Synthesemethoden, insbesondere der Copräzipitierung, erhaltenen Multielementoxidkatalysatoren, ist eine Calcinierung des aus dem erfindungsgemäßen Verfahren erhaltenen Multielementoxidkatalysators prinzipiell nicht erforderlich. Erfindungsgemäß erfolgt daher die Herstellung von Multielementoxidkatalysatoren unter deutlichen milderen Bedingungen als in der Copräzipitation. Im Gegensatz zu dieser gängigen Herstellungsmethode wird im erfindungsgemäßen Verfahren auch eine Anreicherung von Bismut in der katalytisch aktiven Phase vermieden. Gegebenenfalls kann alternativ oder ergänzend zu einer Trocknung dennoch eine Calcinierung im erfindungsgemäßen Verfahren durchgeführt werden.

In einer Ausführungsform des erfindungsgemäßen Verfahrens umfasst das Verfahren daher zusätzlich die Schritte
e) mindestens einmaliges Waschen des aus Schritt d) erhaltenen Multielementoxidkatalysators, und
f) anschließendes Trocknen und/oder Calcinieren des Multielementoxidkatalysators.

Der nach dem erfindungsgemäßen Verfahren erhaltene Multielementoxidkatalysator liegt üblicherweise in Pulverform vor. Für eine industrielle Anwendung ist es vorteilhaft, den pulverförmigen Katalysator nach Zugabe von Form- und Bindemitteln in eine besser handhabbare Form zu bringen. Dies kann durch Aufbringen des Katalysators auf einen Träger, beispielsweise aus Aluminiumoxid, Zirkoniumoxid, Titandioxid oder Siliziumdioxid, oder durch andere formgebende Schritte wie Tablettierung oder Extrusion erfolgen. Die geometrische Form des Trägers ist hierbei nicht einschränkend, sondern richtet sich vielmehr nach den Vorgaben des Reaktors, zum Beispiel Rohrdurchmesser, Länge der Katalysatorschüttung. Der Träger kann daher beispielsweise eine Pyramide, ein Zylinder, ein Sattel, eine Kugel oder ein Vieleck sein, er kann aber auch eine Wand eines Reaktionsraumes sein, beispielsweise als ein Wandreaktor.

Als Bindemittel können diverse Öle, Cellulosen, Polyvinylalkohole, Saccharide, Acrylate, Alkylderivate derselben, Kondensate derselben oder Mischungen davon eingesetzt werden. Bevorzugte Bindemittel sind Acrylate, Polyvinylalkohole, Cellulose, Alkylderivate derselben und Mischungen davon.

Ein geträgerter Katalysator wird beispielsweise durch Aufsprühen entweder einer Suspension des Katalysators in einem geeigneten Lösungsmittel bzw. Lösungsmittelgemisch zusammen mit dem Bindemittel auf den Trägerkörper oder durch Aufsprühen einer Suspension des Katalysators auf einem mit dem Bindemittel befeuchteten Trägerkörper hergestellt. Geeignete Lösungsmittel sind beispielsweise Wasser, Methanol, Ethanol oder ähnliche Verbindungen oder Mischungen davon. Ein Vollkatalysator wird beispielsweise durch Vermischen des Katalysators mit einem vorstehend genannten Bindemittel und anschließender Formgebung hergestellt.

Der durch die jeweilige Formgebung erhaltene Katalysator wird anschließend einer Trocknung und/oder einer Calcinierung zur Vervollständigung der Entfernung der Lösungsmittel und insbesondere der Bindemittel unterworfen.

In einer weiteren Ausführungsform umfasst das erfindungsgemäße Verfahren daher die zusätzlichen Schritte
i) Aufbringen des aus Schritt d), e) oder f) erhaltenen Multielementoxidkatalysators auf einen Träger unter Erhalt eines geträgerten Katalysators,
   oder
ii) Formen des aus Schritt d), e) oder f) erhaltenen Multielementoxidkatalysators unter Erhalt eines Vollkatalysators,
   und
iii) Trocknen und/oder Calcinieren des aus Schritt i) oder ii) erhaltenen Multielementoxidkatalysators.

Untersuchungen der nach dem erfindungsgemäßen Verfahren erhaltenen Multielementoxidkatalysatoren in einem Rastertunnelmikroskop mit energiedispersiver Röntgenspektroskopie (scanning electron microscope mit energy dispersive X-ray spectroscopy, SEM-EDX) haben gezeigt, dass die Katalysatoren Bereiche bzw. Zonen aufweisen, in denen sowohl die als katalytisch aktiv erachteten Elemente Molybdän und Bismut als auch Eisen gemeinsam vorliegen. Es wird vermutet, dass die verbesserten katalytischen Eigenschaften der erfindungsgemäßen Katalysatoren in der Partialgasphasenoxidation von Olefinen auf das gemeinsame Vorliegen der Elemente Molybdän, Bismut und Eisen in Bereichen der Katalysatoren zurückzuführen sind.

Ein weiterer Gegenstand der vorliegenden Erfindung ist daher auch ein Multielementoxidkatalysator der allgemeinen Formel

MoₐBi_{b}Co_{c}Fe_{d}NiₑX_{f}X'gX"ₕX'"ᵢX""ⱼOₓ (I)

mit
- X: = W und/oder P,
- X': = Li, K, Na, Rb, Cs, Mg, Ca, Ba und/oder Sr,
- X": = Ce, Mn, Cr und/oder V,
- X'": = Nb, Se, Te, Sm, Gd, La, Y, Pd, Pt, Ru, Ag und/oder Au,
- X"": = Si, Al, Ti und/oder Zr,
und
- a: = 12,
- b: = 1 bis 4,
- c: = 4 bis 10,
- d: = 1 bis 4,
- e: = 0 bis 4,
- f: = 0 bis 5,
- g: = 0 bis 2,
- h: = 0 bis 5,
- i: = 0 bis 2,
- j: = 0 bis 800,
und
x = eine Zahl, die von der Wertigkeit und Häufigkeit der von Sauerstoff verschiedenen Elemente bestimmt wird,
umfassend die Schritte a) bis d),
der dadurch gekennzeichnet ist, dass er Bereiche aufweist, die Molybdän, Bismut und Eisen gemeinsam enthalten.

In einer Ausführungsform sind die erfindungsgemäßen Multielementoxidkatalysatoren nach dem erfindungsgemäßen Verfahren erhalten und/oder erhältlich.

Die Elemente Molybdän, Bismut und Eisen liegen insbesondere dann gemeinsam in Bereichen bzw. Zonen des erfindungsgemäßen Katalysatoren vor, wenn der pH-Wert in der Hydrothermalsynthese der Katalysatoren 7 +/- 0,5 beträgt. In diesem Fall wird in Schritt b) des erfindungsgemäßen Verfahren ein pH-Wert von 7 +/- 0,5 eingestellt.

In einer Ausführungsform ist der erfindungsgemäße Multielementoxidkatalysator nach dem erfindungsgemäßen Verhalten erhalten und/oder erhältlich, wobei in Schritt b) des erfindungsgemäßen Verfahrens ein pH-Wert von 7 +/- 0,5 eingestellt wird.

In einer Ausführungsform des erfindungsgemäßen Multielementoxidkatalysators bedeuten e bis i jeweils 0.

In einer weiteren Ausführungsform des erfindungsgemäßen Katalysators bedeuten a = 12, b = 1 bis 2, c = 5 bis 8 und d = 2 bis 3.

Im Gegensatz zu den nach gängigen Herstellungsverfahren, insbesondere den nach der Copräzipitation erhaltenen Multielementoxidkatalysatoren müssen die nach dem erfindungsgemäßen Verfahren erhältlichen und/oder erhaltenen Multielementoxidkatalysatoren nicht einer Calcinierung unterzogen werden; vielmehr reicht eine bei niedrigeren als den in einer Calcinierung angewandten Temperaturen durchgeführte Trocknung bereits aus, um den Katalysator in die zum Einsatz in der Katalyse erforderliche Form zu bringen. Eine Calcinierung sowie der Einsatz in der Katalyse wirken sich in der Regel auf die Phasen des betreffenden Katalysators aus, so dass üblicherweise der in der Katalyse eingesetzte Katalysator nicht mehr identisch ist mit dem ursprünglichen, aus dem gängigen Herstellungsverfahren erhaltenen Katalysator oder dem zu Beginn der Katalyse vorliegenden Katalysator. Überraschenderweise wurde nun gefunden, dass der erfindungsgemäße Katalysator und insbesondere der nach dem erfindungsgemäßen Verfahren erhaltene und/oder erhältliche Multielementoxidkatalysator im Wesentlichen phasenstabil sind. Das heißt, dass diese Multielementoxidkatalysatoren während der katalysierten Reaktion wenn überhaupt allenfalls einer geringen Phasenumwandlung unterliegen. Die geringe Phasenumwandlung besteht in einem Intensitätsgewinn für das Raman-Signal bei 875 cm⁻¹ (FeMoO₄) während der katalytischen Reaktion. Im Gegensatz dazu unterliegt ein nach dem Herstellungsverfahren der Copräzipitation, beispielsweise nach dem Verfahren der WO 2007/042369 A1, erhaltener Vergleichskatalysator einer starken Phasenumbildung während der katalysierten Reaktion: Das Raman-Signal für MoO₃ bei 995 cm⁻¹ verschwindet, die Raman-Signale für Bi₂Mo₃O₁₂ bei 814 cm⁻¹ und 900 cm⁻¹ werden schwächer und das Raman-Signal für FeMoO₄ bei 875 cm⁻¹ erscheint.

In einer Ausführungsform ist der erfindungsgemäße Multielementoxidkatalysator, insbesondere der nach der nach dem erfindungsgemäßen Verfahren erhaltene und/oder erhältliche Multielementoxidkatalysator, daher im Wesentlichen phasenstabil, insbesondere während der Verwendung in der Partialgasphasenoxidation von Olefinen.

Bezüglich der Phasenzusammensetzung von hydrothermal synthetisierten Multielementoxidkatalysatoren wird in der Fachliteratur die Auffassung vertreten, dass die eigentliche aktive Phase dieser Katalysatoren die beta-Bismutmolybdat-Phase ist. Die erfindungsgemäßen Multielementoxidkatalysatoren weisen jedoch keine beta-Bismutmolybdat-Phase auf sondern eine alpha-Bismutmolybdat- und eine alpha-Cobaltmolybdat-Phase. Überraschenderweise wurde nun gefunden, dass die erfindungsgemäßen Multielementoxidkatalystoren und insbesondere die nach dem erfindungsgemäßen Verfahren erhaltenen und/oder erhältlichen Multielementoxidkatalysatoren, bevorzugt wenn sie bei einem pH-Wert von 7 +/- 0,5 hergestellt wurden, sowohl eine höhere Aktivität als auch eine höhere Selektivität in der Partialgasphasenoxidation von Olefinen zeigen als Multielementoxidkatalysatoren mit einer beta-Bismutmolybdat-Phase.

In einer Ausführungsform weisen die erfindungsgemäßen Multielementoxidkatalysatoren, insbesondere die nach dem erfindungsgemäßen Verfahren erhaltenen und/oder erhältlichen Multielementoxidkatalysatoren, eine alpha-Bismutmolybdat- und eine alpha-Cobaltmolybdat-Phase auf.

Eine alpha-Bismutmolybdat- und eine alpha-Cobaltmolybdat-Phase weisen insbesondere die nach dem erfindungsgemäßen Verfahren erhaltenen und/oder erhältlichen Multielementoxidkatalysatoren auf, die bei einem pH-Wert von 7 +/- 0,5 in Schritt b) des erfindungsgemäßen Verfahren erhalten wurden.

Die nach dem erfindungsgemäßen Verfahren erhaltenen und/oder erhältlichen Multielementoxidkatalysatoren und die erfindungsgemäßen Multielementoxidkatalysatoren eignen sich für die Partialgasphasenoxidation und die Ammonoxidation von Olefinen.

Im Rahmen der vorliegenden Erfindung werden die Begriffe Partialoxidation, partielle Gasphasenoxidation, Gasphasenpartialoxidation und Partialgasphasenoxidation so verwendet, dass sie Umsetzungen organischer Verbindungen unter reaktiver Einwirkung von Sauerstoff bezeichnen, bei denen die zu oxidierende Verbindung nach beendeter Umsetzung zumindest ein chemisch gebundenes Sauerstoffatom mehr enthält als zuvor. Im Unterschied zu einer vollständigen Oxidation werden durch eine Partialoxidation, eine partielle Gasphasenoxidation, eine Gasphasenpartialoxidation und eine Partialgasphasenoxidation nicht sämtliche der in der zu oxidierenden Verbindungen enthaltenen Kohlenstoffatome zu Kohlenstoffdioxid oxidiert.

Ein weiterer Gegenstand der vorliegenden Erfindung ist daher auch die Verwendung eines nach dem erfindungsgemäßen Verfahren erhaltenen und/oder erhältlichen Multielementoxidkatalysators und/oder eines erfindungsgemäßen Multielementoxidkatalysators in der Partialgasphasenoxidation und/oder in der Ammonoxidation von Olefinen.

Die Partialgasphasenoxidation von Olefinen führt zu ungesättigten Aldehyden und den korrespondierenden ungesättigten Carbonsäuren und die Ammonoxidation von Olefinen führt zu ungesättigten Nitrilen. Die wirtschaftlich bedeutendsten ungesättigten Aldehyde, Carbonsäuren und Nitrile sind C₃- und C₄-Verbindungen. Im Rahmen der vorliegenden Erfindung werden daher die nach dem erfindungsgemäßen Verfahren erhaltenen und/oder erhältlichen Mischoxidkatalysatoren und/oder die erfindungsgemäßen Mischoxidkatalysatoren in der industriellen Herstellung von Acrolein und Acrylsäure durch katalysierte Gasphasenpartialoxidation von Propen, von Methylacrolein und Methacrylsäure durch katalysierte Gasphasenpartialoxidation von iso-Buten (2-Methyl-1-propen) oder tert-Butanol (2-Methyl-2-propanol) und der industriellen Herstellung von Acrylnitril durch Ammmonoxidation von Propen eingesetzt. Die Herstellung dieser Verbindungen erfolgt als heterogen katalysierte Oxidation von Propen oder von iso-Buten bzw. tert-Butanol mit Luft oder Sauerstoff oder als heterogen katalysierte Ammonoxidation von Propen mit Ammoniak und Luft oder Sauerstoff an einem Katalysatorfestbett, das überwiegend einen auf Molybdän- und Bismutoxiden basierenden Mischoxidkatalysator enthält.

In einer Ausführungsform der erfindungsgemäßen Verwendung ist daher das Olefin Propen, iso-Buten und/oder tert-Butanol.

Im Rahmen der vorliegenden Erfindung hat sich gezeigt, dass die nach dem erfindungsgemäßen Verfahren erhaltenen und/oder erhältlichen Multielementoxidkatalysatoren und/oder die erfindungsgemäßen Multielementoxidkatalysatoren deutlich selektiver für die Bildung von ungesättigten Aldehyden sind als für die Bildung von ungesättigten Carbonsäuren.

Bevorzugt handelt es sich daher bei der erfindungsgemäßen Verwendung um die Partialgasphasenoxidation von Olefinen zu ungesättigten Aldehyden. Insbesondere handelt es sich bei der erfindungsgemäßen Verwendung um die Partialgasphasenoxidation von Propen zu Acrolein und/oder die Partialgasphasenoxidation von iso-Buten und/oder tert-Butanol zu Methacrolein.

### Beschreibung der Abbildungen:

- Fig. 1:: Korrelation zwischen dem pH-Wert und der Zusammensetzung der Katalysatoren der Versuche 1 bis 3.
- Fig. 2:: Korrelation zwischen dem pH-Wert und der Zusammensetzung der Katalysatoren der Vergleichsversuche V1 bis V3.
- Fig. 3:: Korrelation zwischen der BET-Oberfläche des hydrothermal hergestellten Katalysators Mo₁₂Bi₁Co₈Fe₃Oₓ, dem pH-Wert in der Hydrothermalsynthese und der Trocknungstemperatur vor der Messung (● = Trocknung bei 320°; ■ = Trocknung bei 150°C).
- Fig. 4:: SEM-EDX-Aufnahme des Multielementkatalysators aus Versuch 1 (die Elemente Bismut Bi, Molybdän Mo und Eisen Fe sind entsprechend der Angabe in der Abbildung eingefärbt).
- Fig. 5:: SEM-EDX-Aufnahme des Multielementkatalysators aus Versuch 2 (die Elemente Bismut Bi, Molybdän Mo und Eisen Fe sind entsprechend der Angabe in der Abbildung eingefärbt).

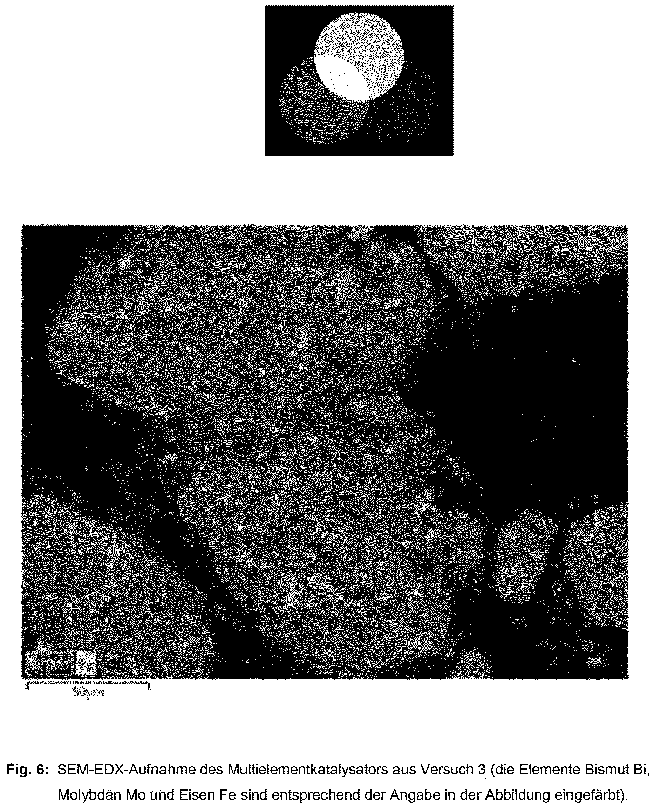

- Fig. 6:: SEM-EDX-Aufnahme des Multielementkatalysators aus Versuch 3 (die Elemente Bismut Bi, Molybdän Mo und Eisen Fe sind entsprechend der Angabe in der Abbildung eingefärbt).
- Fig. 7:: Phasenumwandlungen der hydrothermal synthetisierten (HS) Katalysatoren erhaltenen Katalysators (Raman-Aufnahmen vor der katalysierten Reaktion (gepunktete Linie mit Sternen) und danach (durchgezogene Linie mit Kreuzen)).
- Fig. 8:: Phasenzusammensetzungen des hydrothermal synthetisierten (HS) Katalysators Mo₁₂Bi_{1,5}Co₅Fe₃Oₓ bei unterschiedlichen pH-Werten.
- Fig. 9:: Zeitabhängige Phasenumwandlung des durch Copräzipitation erhaltenen Katalysators Mo₁₂Bi_{1,5}Co₅Fe_{1,8}Oₓ.
- Fig. 10:: Zeitabhängige Phasenumwandlung des hydrothermal bei pH = 7 synthetisierten Katalysators Mo₁₂Bi_{1,5}Co₅Fe₃Oₓ.

### Beispiele

### Beispiel 1: Herstellung von Mo-Bi-Co-Fe-Oxiden

Eine erste Lösung (im Folgenden als Lösung I bezeichnet) wurde hergestellt, indem zunächst gemäß den Angaben der Tabelle definierte Mengen an Bismut(III)nitrat-Pentahydrat, Cobalt(II)nitrat-Hexahydrat und Eisen(III)nitrat-Nonahydrat in 20 mL Salpetersäure (Konzentration 2 M) aufgelöst und für 15 Minuten gründlich gerührt wurden. Parallel dazu wurde eine zweite Lösung (im Folgenden als Lösung II bezeichnet) hergestellt, indem gemäß den Angaben der Tabelle 1 stöchiometrische Mengen an Ammoniumheptamolybdat in 20 mL demineralisiertem Wasser aufgelöst und für 15 Minuten gerührt wurden. In jedem Fall wurden die molaren Mengen der Vorläuferverbindungen von Bismut, Molybdän, Cobalt und Eisen so gewählt, dass sie sich auf insgesamt 20 mmol zusammenzählten. Lösung I und Lösung II wurden in einem Autoklaven-Einleger aus Teflon^{®} vereinigt. In Anschluss wurde der pH-Wert durch tropfenweise Zugabe einer 25%-igen Ammoniak-Lösung mittels eines Titrators (Schott Instruments) eingestellt, und die erhaltene Lösung wurde für weitere 15 Minuten gerührt. Danach wurde der Autoklaven-Einleger mitsamt der darin enthaltenen Lösung in einer Autoklaven aus Stahl mit einem Volumen von 250 mL (Firma Bergerhof) überführt. Dann wurde der Autoklav verschlossen und für 24 Stunden auf eine Temperatur von 180°C erhitzt. Nach dieser Zeitdauer durfte der Autoklav mitsamt Inhalt über einen Zeitraum von weiteren 24 Stunden auf Raumtemperatur abkühlen. Im Anschluss wurde das erhaltene Produkt über einer G4-Glasfritte (Nennweite der Porenweite 10 bis 16 Mikrometer) filtriert. Das feste Produkt wurde dreimal mit 10 mL Wasser und dreimal mit 10 mL Aceton gewaschen. Abschließend wurde das fest Produkt für 48 Stunden bei Raumtemperatur getrocknet und für 5 Stunden bei 320°C kalziniert.

Die Zusammensetzungen der Katalysatoren der Versuche 1 bis 3 und der Vergleichsversuche V1 bis V3 wurden in Beispiel 3 bestimmt.

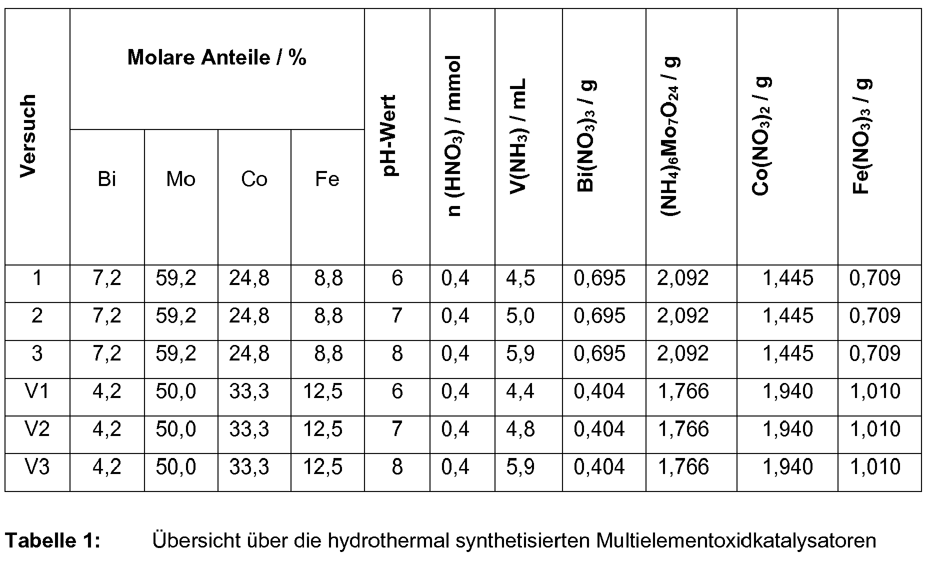

### Beispiel 2: Oberflächenbestimmung der erfindungsgemäßen Katalysatoren

Mittels der BET-Messung wurde die spezifische Oberfläche des bei unterschiedlichen pH-Werten hydrothermal hergestellten Katalysators Mo₁₂Bi₁Co₈Fe₃Oₓ bestimmt. Dafür wurden 100 bis 500 mg des Katalysators für 5 Stunden bei 150°C im Vakuum getrocknet. Die BET-Messungen wurden mit einem BELSORP-Mini II der Firma Rubotherm GmbH durchgeführt. Als Spülgas wurde Helium eingesetzt. Nach der Trocknung wurde die Katalysatorprobe evakuiert und mit flüssigem Stickstoff auf eine Temperatur von 77 K gekühlt. Der Partialdruck des Adsorptionsgases Stickstoff wurde schrittweise erhöht, und es wurde eine Standard-Adsorptionsisotherme im Bereich von p/p₀ = 0 bis 1 aufgezeichnet. Abschließend wurde die Desorptionsisotherme gemessen. Die Bestimmung der BET-Oberfläche erfolgte mit Hilfe der BET-Theorie im Bereich von p/p₀ = 0,05 bis 0,3.

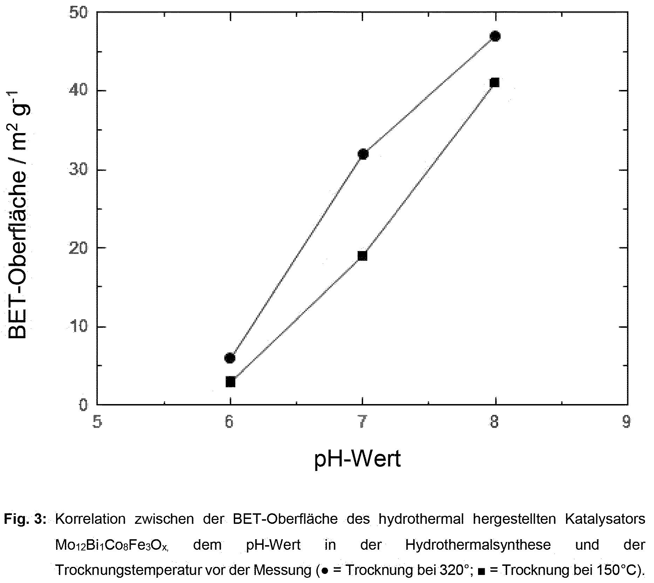

Die ermittelten BET-Oberflächen wurden sowohl mit dem pH-Wert in der Synthese des jeweiligen Katalysators als auch mit den Trocknungstemperaturen korreliert. Abbildung 3 zeigt diese Korrelationen.

Die spezifische Oberfläche der Katalysatoren steigt mit dem bei der Synthese eingestellten pH-Wert an. Eine bei höheren Temperaturen durchgeführte Trocknung führt ebenfalls zu einer größeren spezifischen Oberfläche.

### Beispiel 3: Elementaranalyse der hergestellten Katalysatoren

Die Zusammensetzung der Katalysatoren der Versuche 1 bis 3 und der Vergleichsbeispiele V1 bis V3 wurde mittels optischer Emissionsspektroskopie mit induktiv gekoppeltem Plasma (inductive coupled plasma optical emission spectroscopy, ICP-OES, Typ 720/725-ES von Agilent) durchgeführt. Das Plasma wurde durch einen 40 MHz-Hochfrequenz-Generator erzeugt, und Argon wurde als Plasmagas verwendet. Das Aufschließen von ungefähr 40 mg Probe für die optische Emissionsspektroskopie erfolgte durch Suspendieren der Probe in einer Mischung aus 6 mL konzentrierter Salzsäure, 2 mL konzentrierter Salpetersäure und 1 mL Wasserstoffperoxid und anschließende Behandlung der erhaltenen Mischung in einer Mikrowelle bei 600 Watt für 45 Minuten.

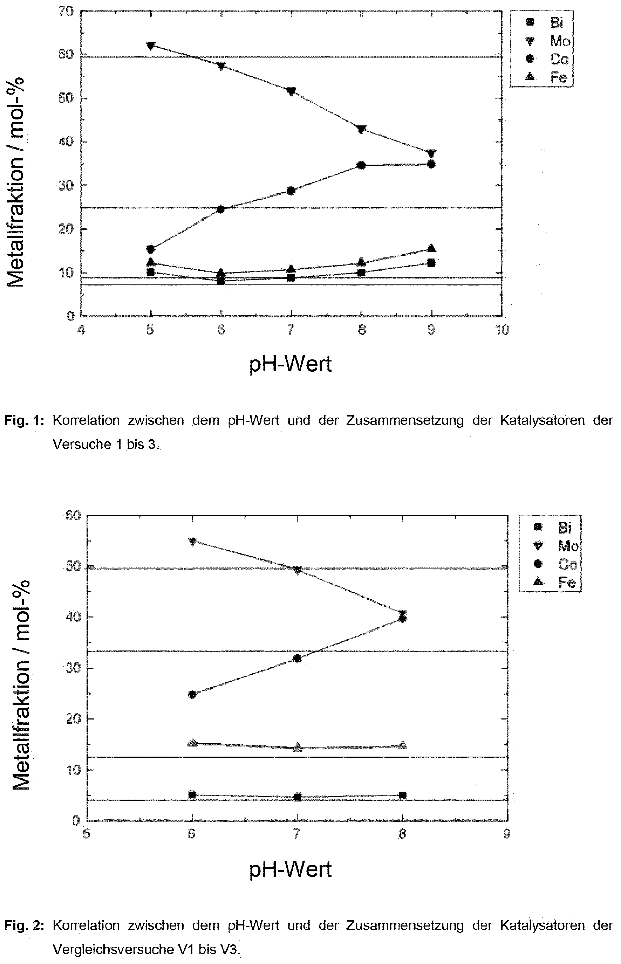

Die für die jeweiligen Katalysatoren bestimmten Metallfraktionen pro Mol wurden mit für die Hydrothermalsynthese eingestellten pH-Werten korreliert. Diese Korrelationen sind in der Abbildung 1 für die Katalysatoren der Versuche 1 bis 3 und in der Abbildung 2 für die Katalysatoren der Vergleichsversuche V1 bis V3 dargestellt.

Der bei pH = 6 hergestellte Multielementoxidkatalysator aus Versuch 1 hatte eine Zusammensetzung von in etwa Mo₁₂Bi₁,₅Co₅Fe₁.₈Oₓ. Ausgehend von pH = 5 nimmt mit steigendem pH-Wert der molare Anteil der katalytischen Komponente Molybdän kontinuierlich von ca. 62 mol-% bis auf ca. 37 mol-% bei pH = 8 ab. Gleichzeitig nimmt mit steigendem pH-Wert der molare Anteil von Cobalt von 15 mol-% bei pH = 5 bis auf 35 mol-% bei pH = 9 zu. Der Anteil von Bismut bzw. Eisen nimmt mit steigendem pH-Wert lediglich um wenige mol-% zu.

Der bei pH = 7 herstellte Multielementoxidkatalysator aus Vergleichsbeispiel V2 hatte eine Zusammensetzung von in etwa Mo₁₂Bi₁Co₈Fe₃Oₓ. Ausgehend von pH = 6 nimmt mit steigendem pH-Wert der molare Anteil der katalytischen Komponente Molybdän kontinuierlich von 55 mol-% bis auf ca. 40 mol-% bei pH = 8 ab. Gleichzeitig nimmt mit steigendem pH-Wert der molare Anteil von Cobalt von 25 mol-% bis auf ca. 40 mol-% bei pH = 8 zu. Der Anteil von Bismut bzw. Eisen bleibt unabhängig vom pH-Wert im Wesentlichen konstant bei ca. 5 mol-% bzw. ca. 15 mol-%.

Die Korrelation zwischen den pH-Werten bei der Hydrothermalsynthese und der jeweiligen Zusammensetzung des Katalysators zeigt, dass der pH-Wert einen großen Einfluss auf die Zusammensetzung des Katalysators hat. Insbesondere der molare Anteil der katalytisch aktiven Komponente Molybdän wird durch den pH-Wert stark beeinflusst.

### Beispiel 4: SEM-EDX-Messungen

Die Oberfläche der Multielementoxidkatalysatoren aus den Versuchen 1 bis 3 wurde in einem Rastertunnelmikroskop mit energiedispersiver Röntgenspektroskopie (scanning electron microscope mit energy dispersive X-ray spectroscopy, SEM-EDX) untersucht.

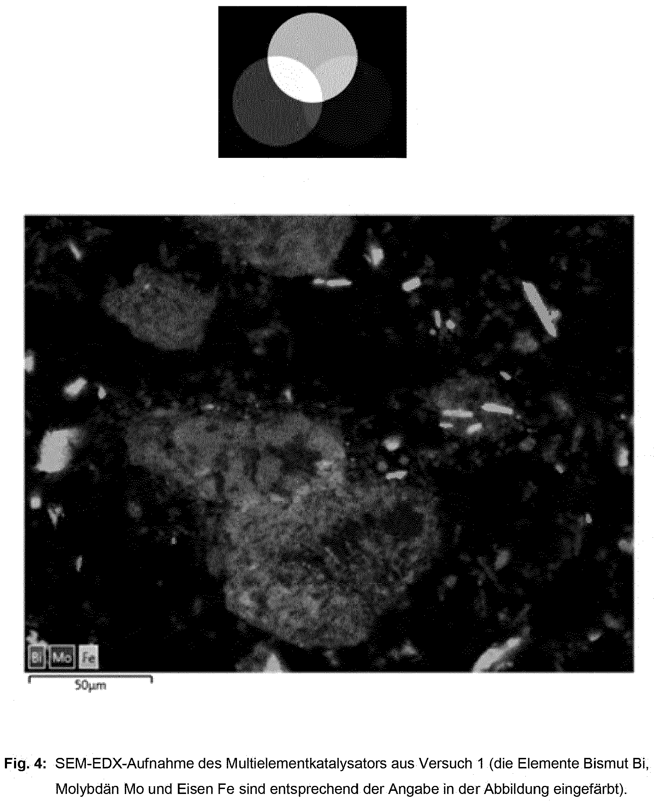

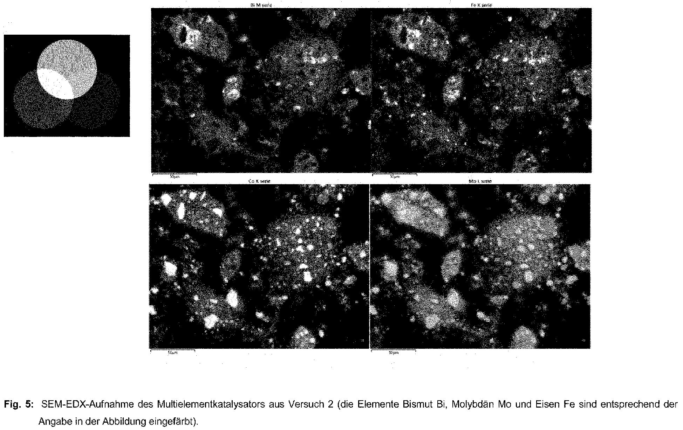

Die mittels SEM-EDX gemachten Aufnahmen der Oberflächen der Multielementoxidkatalysatoren der Versuche 1 bis 3 zeigen die Abbildungen 3 bis 5. In diesen Aufnahmen wurden die Elemente Molybdän Mo, Bismut Bi und Eisen Fe eingefärbt. Bereiche der Katalysatoren in denen alle drei Elemente gemeinsam vorliegen sind - entsprechend der Überlappung der Einfärbungen für die einzelnen Elemente - in den Abbildungen weiß dargestellt. Lediglich der Multielementoxidkatalysator aus dem Versuch 2, hergestellt bei pH = 7, weist weiße Bereiche auf, also Bereiche in denen alle drei Elemente Molybdän, Bismut und Eisen gemeinsam vorliegen.

### Beispiel 5: Phasenbestimmungen

Die Phasen wurden mittels Ramanspektroskopie bestimmt. Dazu wurde ein Reishaw inVia Reflex Spektrometer System der Firma Renishaw ausgestattet mit einem Research Grade Leica DM2500 Mikroskop eingesetzt. Die Proben wurden mit Helium-Neon-Laser mit 17 mW bei 623 nm gemessen. Dazu wurde typischerweise der Spektralbereich von 50 bis 1300 cm⁻¹ aufgezeichnet.

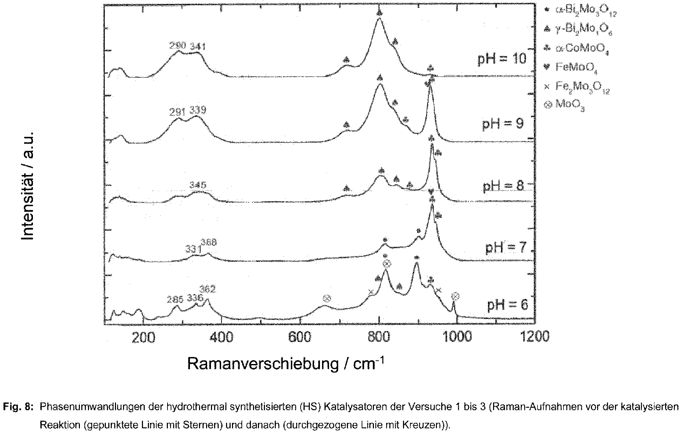

Bei Abbildung 8 wurde der Laserstrahl bei Raumtemperatur mit einem Leica 50x Objektiv auf die Probe fokussiert. Es wurden jeweils mehr als 2000 Spektren über Bereiche von ca. 300 * 300 µm aufgenommen und über diese Fläche gemittelt. Die erhaltenen Raman-Spektren wurden mit mit Hilfe von Literaturwerten den entsprechenden Phasen von Bi, Mo, Co und Fe zugeordnet.

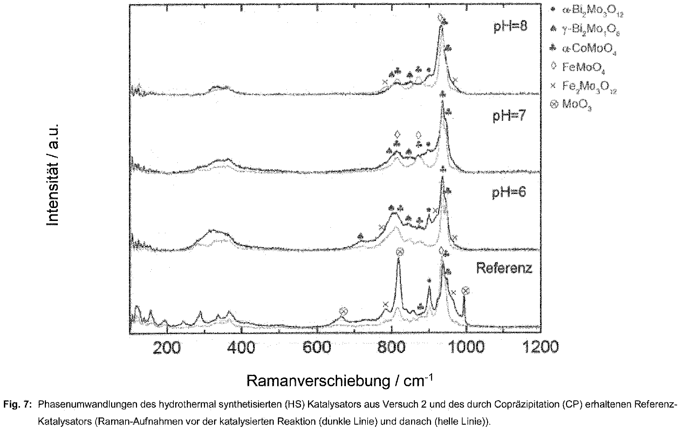

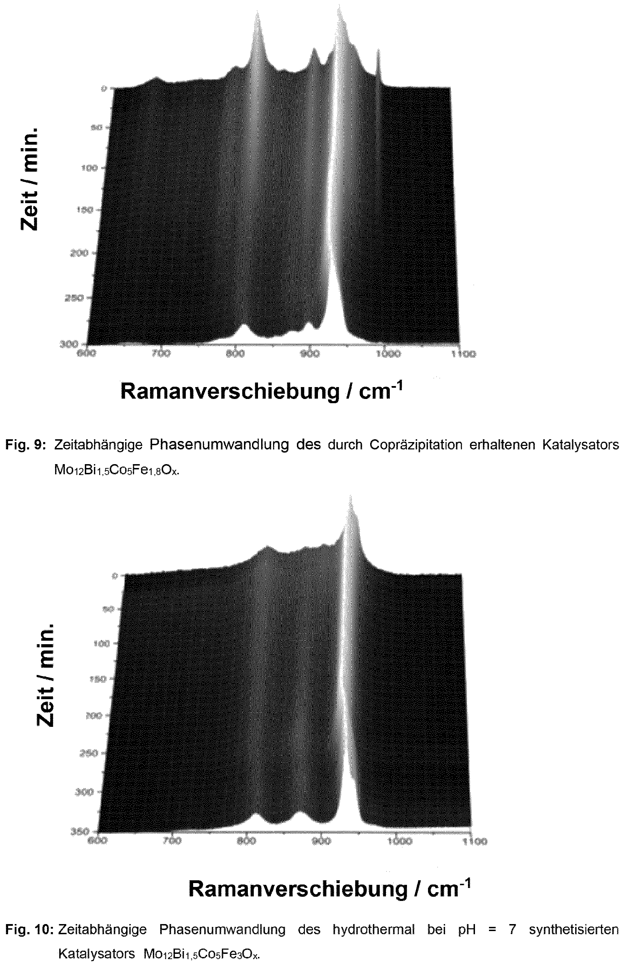

Für die in der Abbildung 7 und den Abbildungen 9 und 10 wiedergegebenen Spektren wurden jeweils 30 mg Katalysator mit einer Partikelgröße von 150 bis 300 µm in eine Quarzglaskapillare mit 2 mm Durchmesser gefüllt. Über diese Quarzglaskapillare wurde ein Gasstrom von 10 mL pro Minute mit der Zusammensetzung N₂/O₂/C₃H₃/H₂ im Verhältnis 72/18/8/8 geleitet. Die Quarzglaskapillare wurde mit einem Gasbläser der Firma Oxford auf eine Temperatur von bis zu 445°C erhitzt. Der Laserstrahl wurde mit Hilfe einer an das Raman-Spektrometer angeschlossenen Video Fibre Optic der Firma Renishaw GmbH auf diese Kapillare fokussiert. Die Spektren in der Abbildung 7 wurden jeweils bei 80°C vor und nach der katalysierten Reaktion aufgenommen. Die Spektren in den Abbildungen 9 und 10 wurden während der katalysierten Reaktion aufgenommen, wobei über den Gasbläser folgendes Temperaturprofil vorgegeben wurde: 180°C (0 min) → 4 K min⁻¹ → 320°C (20 min) → 4 K min⁻¹ → 345°C (46 min) → 4 K min⁻¹ → 370°C (46 min) → 4 K min⁻¹ → 395°C (46 min) → 4 K min⁻¹ → 420°C (23 min) → 4 K min⁻¹ → 445°C (23 min) → -10 K min⁻¹ → 180°C (18 min) → -10 K min⁻¹ → 60°C.

### Beispiel 6: Testung der erfindungsgemäßen Katalysatoren

Die erfindungsgemäßen Katalysatoren nach der Trocknung, ein erfindungsgemäßer Katalysator nach der Calcinierung, ein mittels Copräzipitation gemäß der WO 2007/042369 A hergestellter Katalysator und ein binärer mittels Hydrothermalsynthese Katalysator wurden in der Partialgasphasenoxidation von Propen getestet. Für jeden Test wurde ein Reaktor mit einem Innendurchmesser von 6 mm mit jeweils 800 mg des zu testenden Katalysators einer Siebfraktion von 300 bis 450 µm so befüllt, dass sein Innenraum im Wesentlichen komplett von dem Katalysator ausgefüllt wurde. Das resultierende Katalysatorfestbett wies eine Höhe von 2,7 cm auf. Am oberen und am unteren Ende des Katalysatorfestbetts befand sich jeweils ein Thermoelement, das in Kontakt mit dem Katalysatorfestbett stand. Über eine äußere Wärmezufuhr wurde die Temperatur des Katalysatorfestbetts auf einen Wert von ca. 380°C eingestellt. Jedem mit einem spezifischen Katalysator befüllten Reaktor wurde in drei Versuchen jeweils ein Eduktgasgemisch der Zusammensetzung N₂/O₂/C₃H₆/H₂ im Verhältnis 70/14/8/8 mit einer Flussgeschwindigkeit von 100, 150 und 200 NmL pro Minute zugeführt; bezogen auf die eingesetzte Masse des jeweiligen Katalysators betrug somit die modifizierte Verweilzeit (als Quotient von eingesetzter Masse des Katalysators durch die Flussgeschwindigkeit) für die unterschiedlichen Flussgeschwindigkeiten 0,48 g s ml⁻¹, 0,32 g s ml⁻¹ bzw. 0,24 g s ml⁻¹ Während der Tests wurde die Temperatur des Katalysatorbetts im Wesentlichen konstant bei 380°C gehalten, indem bei Temperaturschwankungen im Katalysatorfestbett die Temperatur der äußeren Wärmequelle entsprechend angepasst wurde. Das aus dem Reaktor austretende Reaktionsgasgemisch wurde über eine beheizte Leitung zu einem Gaschromatographen des Typs Agilent 7890B geleitet.

Der Gaschromatograph war mit zwei Probenschleifen für Permanentgase, insbesondere Stickstoff, Sauerstoff, Kohlenstoffmonoxid und Kohlenstoffdioxid, und für Kohlenwasserstoffe, insbesondere Propen, Acrolein und Acrylsäure, ausgestattet. Die erste Probenschleife bestand aus zwei hintereinander geschalteten Trennsäulen des Typs Hayesep Q von Agilent und einer abschließenden mikrogepackten Säule des Typs Molsieve 5A von Agilent. Die erste Hayesep Q-Trennsäule diente zur Abtrennung der Permanentgase von den Kohlenwasserstoffen, die zweite Hayesep Q-Trennsäule diente zur Auftrennung der Permanentgase: Kohlenstoffdioxid wurde als erstes Gas von der zweiten Hayesep Q-Trennsäule eluiert, und danach wurden die übrigen Permanentgase zusammen von der zweiten Hayesep Q-Trennsäule eluiert und über ein Ventil auf eine finale Trennsäule vom Typ Molsieve 5A geleitet, die zur Trennung der Permanentgase N₂, O₂, CO diente. CO₂ und die von der Molsieve 5A-Trennsäule eluierten Permanentgase wurden mit einem Wärmeleitfähigkeitsdetektor detektiert. Die zweite Probenschleife bestand aus einer Kapillarsäule des Typs HP-FFAP von Agilent. Die von dieser Trennsäule eluierten Verbindungen Propen, Acrolein und Acrylsäure wurden mit einem Flammenionisationsdetektor detektiert. Mit Gasmischungen bekannter Konzentrationen von N₂, CO, CO₂, Propen und Propan sowie einer Gasmischung von N₂ und O₂ und mit Standardlösungen von Propen, Acrolein und Acrylsäure in Methanol wurde der Gaschromatograph kalibriert. Nach dieser Kalibrierung erfolgte die Trennung und Bestimmung der einzelnen Komponente der Reaktionsgasgemische in den einzelnen Katalysatortestungen. Aus den eingesetzten Mengen an Propen und den detektierten Mengen an Propen, Acrolein, Acrylsäure und Kohlenstoffoxiden CO und CO₂ wurden der Propen-Umsatz sowie die Selektivitäten für die Bildung von Acrolein, Acrylsäure und Kohlenstoffoxiden CO und CO₂ ermittelt. Diese Werte sind in den untenstehenden Tabellen 2 bis 4 zusammengefasst.

**Tabelle 2: Übersicht über die katalytischen Eigenschaften der getesteten Katalysatoren bei w/F = 0,48 g s mL⁻¹.**

| **Katalysator** | **Propylen-Umsatz /%** | **Acrolein-Selektivität /%** | **CO + CO₂ Selektivität /%** | **Acrylsäure-Selectivität /%** | **Ofentemperatur /°C** | **T(Kat.) am Eingang /°C** | **T(Kat.) am Ausgang /°C** | **ΔT(Kat) /K** |
|---|---|---|---|---|---|---|---|---|
| AT0928T01T01 | 47,0 | 63,5 | 27,7 | 8,3 | 373 | 395 | 366 | 29 |
| HS-Bi_{1.5}Mo₁₂Co₅Fe₃Oₓ-pH6 | 29,0 | 50,5 | 44,6 | 4,4 | 348 | 363 | 393 | 30 |
| HS-Bii.5Moi2Co5Fe30x-pH7 | 63,4 | 75,9 | 20,9 | 2,8 | 354 | 376 | 384 | 8 |
| HS-Bi_{1.5}Mo_{1.2}Co₅Fe₃Oₓ-pH8 | 52,6 | 59,7 | 25,7 | 14,2 | 370 | 378 | 383 | 5 |
| HS-Bi₁Mo₁Oₓ-pH6 | 18,1 | 79,7 | 19,7 | 0,0 | 380 | 377 | 383 | 6 |

**Tabelle 3: Übersicht über die katalytischen Eigenschaften der getesteten Katalysatoren bei w/F = 0,32 g s mL⁻¹.**

| **Katalysator** | **Propylen-Umsatz /%** | **Acrolein-Selektivität /%** | **CO + CO₂ Selektivität /%** | **Acrylsäure-Selectivität /%** | **Ofentemperatur /°C** | **T(Kat.) am Eingang /°C** | **T(Kat.) am Ausgang /°C** | Δ**T(Kat) /K** |
|---|---|---|---|---|---|---|---|---|
| AT0928T01T01 | 38,0 | 65,7 | 20,2 | 13,8 | 371 | 390 | 370 | 20 |
| HS-Bi_{1.5}Mo₁₂CO₅Fe₃Oₓ-pH6 | 19,6 | 63,3 | 36,3 | 0,0 | 350 | 361 | 397 | 36 |
| HS-Bii.5Moi2Co5Fe30x-pH7 | 56,4 | 77,2 | 18,0 | 4,6 | 353 | 378 | 383 | 5 |
| HS-Bi_{1.5}Mo₁₂Co₅Fe₃Oₓ-pH8 | 41,3 | 69,1 | 21,5 | 9,2 | 369 | 373 | 387 | 14 |
| HS-Bi₁Mo₁Oₓ-pH6 | 12,5 | 94,0 | 5,6 | 0,0 | 381 | 377 | 384 | 7 |

**Tabelle 4: Übersicht über die katalytischen Eigenschaften der getesteten Katalysatoren bei w/F = 0,24 g s mL⁻¹.**

| **Katalysator** | **Propylen-Umsatz /%** | **Acrolein-Selektivität /%** | **CO + CO₂ Selektivität /%** | **Acrylsäure-Selectivität /%** | **Ofentemperatur /°C** | **T(Kat.) am Eingang /°C** | **T(Kat.) am Ausgang /°C** | Δ**T(Kat) /K** |
|---|---|---|---|---|---|---|---|---|
| AT0928T01T01 | 32,0 | 70,5 | 19,0 | 10,1 | 370 | 388 | 368 | 20 |
| HS-Bi_{1.5}Mo₁₂Co₅Fe₃Oₓ-pH6 | 16,2 | 62,8 | 33,0 | 3,4 | 353 | 360 | 400 | 40 |
| HS-Bi_{1.5}Mo₁₂Co₅Fe₃Oₓ-pH7 | 47,5 | 79,3 | 14,9 | 5,6 | 350 | 375 | 385 | 10 |
| HS-Bi_{1.5}Mo₁₂Co₅Fe₃Oₓ-pH8 | 33,8 | 72,5 | 19,0 | 8,3 | 369 | 370 | 390 | 20 |
| HS-Bi₁Mo₁Oₓ-pH6 | 10,9 | 96,0 | 2,7 | 0,0 | 381 | 377 | 384 | 7 |

## Patentansprüche

1. Verfahren zur Herstellung eines Multielementoxidkatalysators der allgemeinen Formel (I)
MoₐBi_{b}Co_{c}Fe_{d}NiₑX_{f}X'_{g}X"ₕX'"ᵢX""ⱼOₓ (I)
mit
X = W und/oder P,
X' = Li, K, Na, Rb, Cs, Mg, Ca, Ba und/oder Sr,
X" = Ce, Mn, Cr und/oder V,
X'" = Nb, Se, Te, Sm, Gd, La, Y, Pd, Pt, Ru, Ag und/oder Au,
X"" = Si, Al, Ti und/oder Zr,
und
a = 12,
b = 1 bis 4,
c = 4 bis 10,
d = 1 bis 4,
e = 0 bis 4,
f = 0 bis 5,
g = 0 bis 2,
h = 0 bis 5,
i = 0 bis 2,
j = 0 bis 800,
und
x = eine Zahl, die von der Wertigkeit und Häufigkeit der von Sauerstoff verschiedenen Elemente bestimmt wird, umfassend die Schritte
a) Bereitstellen einer Mischung aus einer wässrigen Lösung und/oder einer wässrigen Suspension von Vorläuferverbindungen der im herzustellenden Multielementoxidkatalysator enthaltenen Elemente in der zur Erzielung der Stöchiometrie gemäß der allgemeinen Formel erforderlichen Menge,
b) Einstellen der aus Schritt a) erhaltenen Mischung auf einen pH-Wert zwischen 6 +/-0,5 und 8 +/- 0,5,
c) Umsetzen der Ausgangsverbildungen unter im Wesentlichen solvothermalen Reaktionsbedingungen unter Bildung eines Multielementoxidkatalysators der allgemeinen Formel (I), und
d) Abtrennen des Multielementoxidkatalysators von der wässrigen Lösung und/oder Suspension.

2. Verfahren gemäß Anspruch 1, wobei in Schritt b) ein pH-Wert von 7 +/- 0,5 eingestellt wird.

3. Verfahren gemäß Anspruch 1 oder 2, wobei der Schritt c) bei einer Temperatur von mehr als 100°C bis 600°C durchgeführt wird.

4. Verfahren gemäß Anspruch 3, wobei der Schritt c) bei einer Temperatur von mehr als 100°C bis 400°C durchgeführt wird.

5. Verfahren gemäß einem beliebigen der Ansprüche 1 bis 4, wobei der Schritt c) für eine Zeitdauer von 6 +/- 0,5 Stunden bis zu 48 +/- 0,5 Stunden durchgeführt wird.

6. Verfahren gemäß einem beliebigen der Ansprüche 1 bis 5, zusätzlich umfassend die Schritte
e) mindestens einmaliges Waschen des aus Schritt d) erhaltenen Multielementoxidkatalysators, und
f) anschließendes Trocknen und/oder Calcinieren des Multielementoxidkatalysators.

7. Verfahren gemäß einem beliebigen der Ansprüche 1 bis 6, zusätzlich umfassend die Schritte
i) Aufbringen des aus Schritt d), e) oder f) erhaltenen Multielementoxidkatalysators auf einen Träger unter Erhalt eines geträgerten Katalysators,
oder
ii) Formen des aus Schritt d), e) oder f) erhaltenen Multielementoxidkatalysators unter Erhalt eines Vollkatalysators,
und
ii) Trocknen und/oder Calcinieren des aus Schritt i) oder ii) erhaltenen Multielementoxidkatalysators.

8. Multielementoxidkatalysator der allgemeinen Formel
MoₐBi_{b}Co_{c}Fe_{d}NiₑX_{f}X'_{g}X"ₕX'"ᵢX""ⱼOₓ (I)
mit
X = W und/oder P,
X' = Li, K, Na, Rb, Cs, Mg, Ca, Ba und/oder Sr,
X" = Ce, Mn, Cr und/oder V,
X'" = Nb, Se, Te, Sm, Gd, La, Y, Pd, Pt, Ru, Ag und/oder Au,
X"" = Si, Al, Ti und/oder Zr,
und
a = 12,
b = 1 bis 4,
c = 4 bis 10,
d = 1 bis 4,
e = 0 bis 4,
f = 0 bis 5,
g = 0 bis 2,
h = 0 bis 5,
i = 0 bis 2,
j = 0 bis 800,
und
x = eine Zahl, die von der Wertigkeit und Häufigkeit der von Sauerstoff verschiedenen Elemente bestimmt wird, umfassend die Schritte,
der **dadurch gekennzeichnet ist, dass** er Cluster von Molybdän, Cobalt und Eisen aufweist.

9. Multimetalloxidkatalysator gemäß Anspruch 8, erhalten und/oder erhältlich nach einem Verfahren gemäß einem beliebigen der Ansprüche 1 bis 7.

10. Multielementoxidkatalysator gemäß Anspruch 9, wobei in Schritt b) des Verfahrens ein pH-Wert von 7 +/- 0,5 eingestellt wird.

11. Multielementoxidkatalysator gemäß einem beliebigen der Ansprüche 8 bis 10, wobei e bis i jeweils 0 bedeuten.

12. Multielementoxidkatalysator gemäß einem beliebigen der Ansprüche 8 bis 11, wobei a = 12, b = 1 bis 2, c = 5 bis 8 und d = 2 bis 3 bedeuten.

13. Multielementoxidkatalysator gemäß einem beliebigen der Ansprüche 8 bis 12, wobei der Multielementoxidkatalysator eine alpha-Bismutmolybdat- und eine alpha-Cobaltmolybdat-Phase aufweist.

14. Verwendung eines nach einem Verfahren gemäß einem beliebigen der Ansprüche 1 bis 7 erhaltenen und/oder erhältlichen Multielementoxidkatalysators und/oder eines Multielementoxidkatalysators gemäß einem beliebigen der Ansprüche 8 bis 13 in der Partialgasphasenoxidation und/oder in der Ammonoxidation von Olefinen.

15. Verwendung gemäß Anspruch 14, wobei das Olefin Propen, iso-Buten und/oder tert-Butanol ist.
